(19)

Europäisches
Patentamt

European
Patent Office

Office européen
des brevets

(11) **EP 2 793 863 B1**

(12) **EUROPEAN PATENT SPECIFICATION**

(45) Date of publication and mention
of the grant of the patent:
**25.07.2018 Bulletin 2018/30**

(21) Application number: **12816095.9**

(22) Date of filing: **24.12.2012**

(51) Int Cl.:
*A61K 9/14* *(2006.01)*   *A61K 47/12* *(2006.01)*
*A61K 47/18* *(2017.01)*   *A61K 47/26* *(2006.01)*
*A61K 39/395* *(2006.01)*

(86) International application number:
**PCT/GB2012/053265**

(87) International publication number:
**WO 2013/093524 (27.06.2013 Gazette 2013/26)**

(54) **METHOD FOR PREPARING AMORPHOUS PRECIPITATED PROTEIN PARTICLES**

VERFAHREN ZUR HERSTELLUNG VON AMORPHEN AUSGEFÄLLTEN PROTEINTEILCHEN

PROCÉDÉ DE PRÉPARATION DE PARTICULES PROTÉIQUES PRÉCIPITÉES AMORPHES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.12.2011   GB 201122408**

(43) Date of publication of application:
**29.10.2014   Bulletin 2014/44**

(73) Proprietor: **University of Strathclyde
Glasgow G1 1XQ (GB)**

(72) Inventors:
• **VOS, Jan
  Wigtownshire DG8 7BB (GB)**

• **MACLEOD, Andrew John
  Glasgow G53 6UL (GB)**
• **MOORE, Barry Douglas
  Glasgow G63 9QE (GB)**

(74) Representative: **HGF Limited
Delta House
50 West Nile Street
Glasgow G1 2NP (GB)**

(56) References cited:
**WO-A2-2008/132439**

**Description**

**Field of the Invention**

The present invention relates to the provision of a method for preparing dry protein formulations comprising amorphous precipitated protein particles. In particular to a method provides dry protein formulations which can be reconstituted to provide clear, foam free concentrated protein solutions.

**Background of the Invention**

Therapeutic proteins such as monoclonal antibodies are important drugs for the bio-pharmaceutical industry and there are many therapeutic proteins in development, targeted at a wide range of indications. Typically marketed therapeutic proteins are administered parenterally as solutions and treatment may be administered to a subject in hospital via infusion or via injection from a healthcare professional or else be self-administered.

Therapeutic proteins that show poor stability in solution are often stabilized in the dry state. The stabilising effects may vary from protein to protein but can include reducing mobility, increasing conformational stability and preventing or reducing water catalyzed degradation pathways.

When proteins are stored in the dry state as a dry protein formulation, such as a lyophilized powder or cake they most commonly need to be redissolved back into an aqueous diluent before they can be administered to the patient as a solution. The formation of a protein solution on solubilizing a dry protein formulation by addition of a suitable quantity of a diluent, such as water for injection, is generally termed reconstitution. The reconstitution process, beginning with addition of diluent, typically transforms a dry protein formulation from a powder or cake into a solution of the protein. Preferably on completion of the reconstitution process the formed protein solution will be optically clear or else opalescent, but it should not contain any visible particles. This reconstitution process should be completed quite rapidly so that the patient does not have to wait an unreasonable time for the drug to be administered.

To be suitable for administration to patients high protein concentration solutions also need to comprise only pharmaceutically acceptable excipients and to fulfill other criteria such as retention of protein integrity, syringability and acceptable osmolality for injection. There is therefore a need for methods that can produce stable dry powder formulations fulfilling these criteria which can be also be reconstituted in a reasonable time, without formation of foam and with minimal manual intervention.

In order to be suitable for use in preparing high concentration solutions for delivery via parenteral administration, it is desirable that dry protein formulations: contain only parenterally approved excipients; can be reconstituted over a reasonable time period; show suitable retention of protein integrity and bioactivity following processing and high resistance to degradation on storage; produce highly concentrated protein solutions on reconstitution exhibiting good clarity and minimal changes to the protein aggregation state to that present prior to protein drying. To avoid the dose of protein being painful to inject it is also highly desirable that dry protein formulations produce highly concentrated protein solution in which the concentration of excipients present provide a solution having an osmolality of less than about 800 mOsmol/kg and preferably less than about 600 Osmol/kg when reconstituted at the protein concentration to be administered. It is also important that the dry protein formulations produce highly concentrated protein solutions which exhibit good syringability such that they can be injected using an appropriate bore hypodermic needle, such as 27G or preferably narrower using a reasonable speed and force. This may be determined by measuring the actual glide force through the target syringe and needle. In order for the solution to be injectable by patients the glide force should normally be less than 30N and preferably less than 15N.

Some or all of these requirements may be satisfied by dry protein formulations based on lyophilized powders. However, when targeting very high protein concentrations in the range of from about 140mg/ml to about 350 mg/ml it becomes increasingly difficult to achieve all these requirements using a lyophilized preparation and furthermore such preparations typically exhibit longer than desired reconstitution times.

Generally it is desirable to use around a 3-fold greater mass of excipient than protein to prepare lyophilized formulations which fully protect the protein integrity and exhibit good storage stability. However, in order to meet osmolality requirements for highly concentrated solution the mass of excipient in the dry formulation will typically need to be equal to or less than that of the mass of protein and so it may be very difficult to prepare lyophilized powders that are suitable. It has been disclosed previously that dry formulations based on protein coated microcrystals (PCMCs) can be prepared that can be rapidly reconstituted to moderately high concentrations of 72 mg/ml. Thus, in Example 15 of WO2008132439A2 at pages 86 to 87, the excipient glycine was exemplified as the core crystalline material to prepare PCMC dry powders with a protein loading of 29 %w/w that could be dissolved to form solutions with a protein concentration of around 72 mg/ml. However, in these experiments no account was taken of the osmolality of the solutions produced. Glycine has a relatively low molecular weight (75 Da) and on reconstitution of these particles was present in solution at 100 mg/ml giving an expected osmolality of over 1300 mOsmol/kg. A solution with such a high osmolality would be expected to result in unacceptable pain if delivered to a patient. Further it would not be possible to form more highly concentrated solutions, such as greater than 140 mg/ml, with such formulations without producing solutions which exhibited unacceptably high osmolality. It is clear therefore that alternate methods are needed for preparation of dry formulations that can be reconstituted rapidly to very high concentrations while also meeting the above requirements for parenteral delivery. Improved methods for reconstituting such formulations are also required.

The presence of visible or sub-visible particles on reconstitution may be indicative that degradation processes leading to the formation of protein aggregates have occurred during one or more of the product manufacturing steps, such as filling, freezing, or drying, or during post-manufacture shipping and storage, or else during the reconstitution process itself. It should be noted that great care is taken during the development of marketed therapeutic proteins products to ensure that the risk of producing particles during the manufacture, shipping and storage is minimised. This is because particles present in an administered solution of a therapeutic protein, particularly those that contain denatured protein, are likely to significantly increase the risk of a patient developing an undesirable immune response towards the protein drug. Problems with immunogenicity may include generation of anti-drug antibodies that neutralize or enhance the clearance of the therapeutic protein or else lead to accumulation of the drug.

The specific process of reconstituting a dry protein formulation is known to carry risks of causing aggregation of the protein which may result in formation of visible or sub-visible particles. Thus, it is well known in the art that shaking or vigorously agitating a protein/diluent mixture can result in a poorly reconstituted product possibly due to shear stresses and/or the production of bubbles which can both cause protein denaturation or unfolding. Thus, commonly the reconstitution instructions provided in the drug product insert of a therapeutic protein, specifically state "do not shake". Protein solutions that have been shaken or agitated too vigorously typically contain a layer of persistent foam in which air bubbles, released during reconstitution of the dry protein formulation powder or else formed directly by shaking or agitation, are stabilized by the presence of denatured protein.

Whilst the reconstitution instructions provided for dry protein formulations vary in detail from protein to protein the following examples of reconstitution steps-in-common are to be found in the product inserts of the following drugs Synagis® (palivizumab), Herceptin® (trastuzmab), Fuzeon® (enfuvirtide), and Xolair® (omalizumab) which are supplied as dry products in vials: manual reconstitution; swirling (gentle) or rolling (gentle); no shaking; avoidance of foam; clear or opalescent solutions; no particulates.

Thus, the reconstitution of dry powder formulations of therapeutic protein is typically carried out by hand and relies on the skill and experience of the responsible person to ensure the process is reproducibly carried out without degradation of the protein. In order to achieve full reconstitution in a reasonable time, such as less than 30 minutes, it is often stated that it is necessary to "gently swirl the vial". However, if this is done incorrectly it can lead to the formation of "excessive foaming" with the possibility that some small fraction of the protein has been degraded. The person responsible for reconstituting a dry protein formulation has therefore to apply the appropriate technique to achieve gentle swirling while also judging whether or not the level of foam produced is excessive or not. Furthermore, if on administration a particular therapeutic protein solution does generate an immune response there is a risk that the patient will henceforth no longer obtain any therapeutic benefit from that drug.

There is therefore a clear need to develop improved reconstitution methods for dry protein formulations that reduce or obviate the risk of inadvertently administering a poorly reconstituted therapeutic protein.

It is an object of the present invention to provide methods of preparing pharmaceutically acceptable formulations containing large doses of therapeutic protein suitable for reconstitution which: reduce or obviate the risk of inadvertently administering a poorly reconstituted therapeutic protein; have acceptable stability as dry powder formulations; produce solutions containing protein of high integrity; produce solutions having good syringability; produce solutions having acceptable osmolality; can be reconstituted in a reasonable time; can be reconstituted without formation of foam; can be reconstituted with minimal manual intervention.

It is a further object of the present invention to provide methods for faster reconstitution of dry protein formulations to high concentration for parenteral delivery that reduce or obviate the risk of inadvertently administering a poorly reconstituted therapeutic protein.; produce stable dry powder formulations; produce solutions containing protein of high integrity; produce solutions having good syringability; produce solutions having acceptable osmolality; that can be reconstituted in a reasonable time; that can be reconstituted without formation of foam; that can be reconstituted with minimal manual intervention.

It is also an object of the present invention to provide methods that can achieve reconstitution of a dry protein formulation to a highly concentrated protein solution in a reduced/reasonable time, without formation of foam and with minimal manual intervention.

## Summary of the Invention

The present disclosure provides a method for the preparation of dry amorphous precipitated protein formulations suitable for reconstitution into highly concentrated protein solutions via:

i) preparation of an aqueous solution for precipitation comprising:

a) a protein at a mass concentration of greater than about 50 mg/ml;
b) an excipient of molecular mass of greater than about 170 Da at a mass concentration of from about 40% to about 95 % relative to that of the protein;
c) a buffer;
d) a cationic precipitation stabilizing additive at a molar concentration of less than about 50mM and/or an anionic precipitation stabilizing additive at a molar concentration of less than about 50mM;
e) optionally additional surfactants, additional buffers or salts,

ii) combining or mixing the aqueous solution with a greater than 12 fold larger volume of a protic organic solvent at a temperature in the range of from about 15°C to about 50°C, to coprecipitate the solid components of the aqueous solution, resulting in formation of a suspension of amorphous protein containing particles, and
iii) isolating the precipitated amorphous protein containing particles from the suspension in the form of a dry powder.

The present disclosure additionally provides a method of the preparation of dry protein formulations suitable for reconstitution into highly concentrated protein solutions via:

(i) preparation of an aqueous solution for precipitation comprising:

a) a protein at a mass concentration of greater than about 50 mg/ml;
b) an excipient of molecular mass of greater than about 170 Da at a mass concentration of about 40% to about 95 % relative to that of the protein;
c) a cationic precipitation stabilizing additive at a molar concentration of less than about 50mM;
d) an anionic precipitation stabilizing additive at a molar concentration of less than about 50mM;
e) optionally additional surfactants, buffers or salts,

(ii) combining or mixing the aqueous solution with a greater than 12 fold larger volume of a protic organic solvent at a temperature in the range of from about 15°C to about 50°C to coprecipitate the solid components of the aqueous solution resulting in formation of a suspension of amorphous protein containing particles, and

(iii) isolating the dry amorphous protein containing particles from the suspension in the form of a dry powder.

The present invention provides a method for preparing preferred amorphous dry precipitated protein particles comprising comprise protein, sucrose, arginine and glutamic acid or lactobionic acid.
The method for preparing amorphous dry precipitated particles that comprise protein, sucrose, arginine and glutamic acid or lactobionic acid which can be reconstituted to form an aqueous solution suitable for subcutaneous administration of said protein at a concentration of greater than 140 mg/ml protein, comprises the following steps:

(i) preparation of an aqueous solution for precipitation comprising:

(a) a protein at a mass concentration of greater than about 50 mg/ml;

(b) sucrose at a mass concentration between about 30% to about 95 % of the mass concentration of the protein;

(c) arginine at a molar concentration of less than about 50 mM;

(d) glutamic acid or lactobionic acid at a molar concentration of less than about 50 mM;

(e) optionally a surfactant;

(ii) combining the aqueous solution for precipitation with a 12 fold or larger volume of one or more protic organic solvents at a temperature in the range of from about 15°C to about 50°C to produce a miscible solvent mixture and suspension of amorphous precipitated particles which each comprise a mixture of protein, sucrose, arginine and glutamic acid or lactobionic acid; and
(iii) concentrating the suspension and/or removing the remaining solvent to isolate the dry amorphous precipitated particles comprising protein.

Any suitable methods for isolating precipitated protein particles in the form of dry powder, or for concentration of the suspension and/or removal of the remaining solvent, many be used in accordance with the methods for the preparation of particles detailed herein. Preferred methods for isolation, concentration and/or solvent removal for use herein are supercritical fluid extraction and/or vacuum drying or air drying.

According to a yet further aspect the present invention provides pharmaceutically acceptable formulations containing highly concentrated or very highly concentrated solutions of therapeutic protein suitable for delivery via subcutaneous administration wherein said formulations are prepared via reconstitution of a dry protein formulation according to novel reconstitution methods as defined hereinafter.

## Description

Cationic and/or anionic stabilizing additives for use in the formulations of the invention can each be independently present at molar concentrations of less than about 50mM or preferably less than about 25 mM.

The mass concentration of the protein as used herein is defined as the mass of the particular protein that is present per unit volume of the aqueous solution it is dissolved in and herein is expressed in units of mg/ml.

Any protein capable of being formulated as a dry formulation may be used in the preparation of dry amorphous precipitated proteins in accordance with the methods herein. In particular proteins known in the art for the preparation of: lyophilized protein powders or cakes; precipitated protein powders or cakes; vacuum dried powders or cakes; air-dried powders or cakes; spray dried powders or cakes; and supercritical fluid dried powders or cakes are suitable for use herein.

Any suitable buffers, salts, surfactants or mixtures thereof as are known in the art for utility in conjunction with dry protein formulations may be used herein. Preferred additives are parenterally approved surfactants such as polysorbates including Tween 80 and/or antioxidants.

Any suitable cationic and anionic precipitation stabilising additives, polar organic solvents and suitable excipients may be used in any of the methods for the preparation of amorphous dry precipitated protein particles as detailed herein.

Preferred polar organic solvents for use herein are protic organic solvents such as alcohols and diols and mixtures thereof, including isobutanol, isopropanol and 2-methyl-2,4-pentane diol. The most preferred protic organic solvent for use herein is isobutanol. This is because it appears to promote formation of amorphous precipitated particles comprising sucrose and also is likely to become widely available as a green fuel which will provide the additional advantage of lowering manufacturing costs. Thus the present invention provides methods for the preparation of any of the dry formulations as detailed herein wherein the organic solvent is: a protic organic solvent or mixture of protic organic solvents; a protic organic solvent independently selected from alcohols and diols and mixtures thereof; a protic organic solvent independently selected from isobutanol, isopropanol and/or 2-methyl-2,4-pentane diol and mixtures thereof; the organic solvent is isobutanol.

The preferred cationic precipitation stabilising additive for use herein is arginine. Preferred anionic precipitation stabilising additives are independently selected from: glutamic acid; and lactobionic acid. These preferred precipitation stabilising additives provide good protein integrity, have higher molecular weight and are parenterally approved. To ensure the reconstituted solution remains within acceptable osmolality limits each of the precipitation stabilising additives should preferably be present in the aqueous solution for precipitation at a molar concentration of less than about 50 mM, and preferably less than about 25 mM. Thus the present invention provides methods for the preparation of any of the dry formulations as detailed herein wherein the each of the precipitation stabilising additives is present in the aqueous solution for precipitation at a molar concentration of less than about 50 mM, preferably less than about 25 mM. A particular advantage of using coprecipitation to prepare dry protein formulations is that the protein concentration present in the precipitated aqueous solution can if desired be much lower than the target protein concentration in the reconstituted solution. Thus using the methods herein it is possible to use protein solutions with a mass concentration in the range of from about 30 to about 140 mg/ml, preferably greater than about 50mg/ml, more preferably greater than about 80mg/ml to prepare dry protein formulations that can be reconstituted to provide protein concentrations in the range of from about 140 to about 350mg/ml. Manufacture of aqueous solutions of lower protein concentration is much more straightforward and minimizes potential problems with reversible and irreversible aggregation.

Suitable proteins for use in herein include peptides <5 KDa, small proteins 5-50 KDa, medium proteins, 50-200 KDa and large proteins >200 KDa. Preferred proteins include: therapeutic proteins, such as those developed for chronic conditions such as arthritis, Crohn's disease and Alzheimer's; diagnostic proteins. Preferred proteins will typically need to be delivered at high therapeutic doses such as greater than 1 mg/kg or greater than 5 mg/kg or greater than 10 mg/kg. Preferred proteins are therefore medium or large proteins. A class of preferred proteins are monoclonal antibodies and conjugates thereof such as drug-protein conjugates and fusion proteins. Proteins that may need to be concentrated and formulated in a closed loop and rapidly are also preferred such as radiolabelled proteins.

Any one of, or combination of the following therapeutic or diagnostic proteins prepared as a dry powder formulation may be used in accordance with the reconstitution method of the present invention as detailed herein: antibodies;

non-antibody proteins; immunoglobulins; immunoglobulin-like proteins; growth factors; fusion proteins, chimeric proteins, enzymes; hormones; cytokines; Fc-derivatised proteins or drugs; and recombinant antigens. Suitable antibodies may be polyclonal, monoclonal, native, recombinant, human, humanized, chimeric, multispecific or single chain. Immunoglobulins from classes IgA, IgD, IgE, IgG and IgM may be used. Suitable IgG may be of any isotype including IgG1, IgG2, IgG2$\Delta$a, IgG3, and IgG4. Antibody-drug conjugates may also be used. Derivatives of antibodies may also be used and these include the antigen-binding portion produced by recombinant DNA techniques or by enzymatic or chemical cleavage of intact antibodies. Antigen-binding portions include, inter alia, Fab, Fab', F(ab').sub.2, Fv, dAb, and complementarity determining region (CDR) fragments, single-chain antibodies (scFv), chimeric antibodies, diabodies and polypeptides that contain at least a portion of an immunoglobulin that is sufficient to confer specific antigen binding to the polypeptide.

Analogs of naturally occurring proteins may be included such as polypeptides with modified glycosylation, polypeptides without glycosylation (unglycosylated). Derivatives of naturally occurring or analog polypeptides which have been chemically modified, for example, to attach water soluble polymers (e.g., pegylated), radionuclides, or other diagnostic or targeting or therapeutic moieties) may also be included.

Marketed dry protein formulations which may benefit from the described centrifuge reconstitution process include Synagis® (palivizumab), Herceptin® (trastuzmab), Fuzeon® (enfuvirtide), Xolair® (omalizumab), Raptiva® (efalizumab), and Ilaris® (canakinumab).

An additional advantage of using the precipitation methods herein for the preparation of amorphous dry protein formulations is that polysorbates and other surfactants may be removed or reduced significantly by dissolution into the protic organic solvent. Removal or reduction of surfactant during the process for making dry protein formulations may be advantageous for stability in the dry state. In addition to lowering the potential for foaming polysorbates are reported to undergo autooxidation, cleavage at the ethylene oxide subunits and hydrolysis of the fatty acid ester bond. Autooxidation results in hydroperoxide formation, side-chain cleavage and eventually formation of short chain acids such as formic acid all of which could influence the stability of a biopharmaceutical product.

As detailed herein present invention additionally provides a method for reconstitution of dry protein formulations comprising:

i) transfer of a dry protein formulation into a suitable reconstitution vessel, or preparation of a dry protein formulation within a suitable reconstitution vessel;

ii) addition of a suitable quantity of an aqueous diluent to the reconstitution vessel;

iii) centrifugation of the reconstitution vessel at a suitable relative centrifugal force for sufficient time to obtain complete or near reconstitution of the dry protein formulation into the aqueous diluent and to produce a protein solution that exhibits minimal foaming;

wherein the order of steps (i) and (ii) may be reversed or combined providing for the transfer of diluent to the vessel followed by addition of dry protein, and providing for the transfer of a preformed mixture of dry protein and diluent to the vessel; and
wherein the dry protein formulation is prepared according to the method according to the present invention.

Where the dry protein is added to the diluent in the reconstitution vessel the present invention provides a method for the reconstitution of dry protein formulations comprising:

i) addition of a suitable quantity of an aqueous diluent to a suitable reconstitution vessel;

ii) transfer of dry protein formulation into the reconstitution vessel; or preparation of a dry protein formulation within the reconstitution vessel; and

iii) centrifugation of the reconstitution vessel at a suitable relative centrifugal force for sufficient time to obtain complete or near complete reconstitution of the dry protein formulation into the aqueous diluent and to produce a protein solution that exhibits minimal foaming; and

wherein the dry protein formulation is prepared according to the method according to the present invention.

The reconstituted solution provided by the method of the present invention is preferably homogeneous, optically clear, and free from visible particles.

The inventors have recognized that although the above method of preparing amorphous dry precipitated protein particles solves many of the problems associated with preparing highly concentrated proteins solutions for parenteral administration there remains an additional problem which is most evident when very high protein concentrations are targeted - the reconstitution time. This may be greater than 30 minutes (min) at high protein concentration (>140 mg/ml) and greater than 60 min at very high protein concentration (200 mg/ml). Reconstitution times longer than 30 min are undesirable for patients or clinicians.

According to a yet further aspect the present invention provides a new method for reconstituting amorphous dry precipitated proteins particles. Advantageously this new method provides reconstituted protein solutions from dry protein formulations within highly desirable time-frames. According to this further reconstitution method reconstituted protein solutions can be typically be prepared in less than about 30 minutes, preferably less than about 20 minutes, preferably between about 10 and about 15 minutes, more preferably between about 5 and about 10 minutes, and especially less than about 5 minutes.

Optionally on removal of the reconstitution vessel from the centrifuge a gentle mixing process can be applied to the solution to ensure homogeneity. Such, gentle mixing will remove any concentration gradients that may exist following centrifugation and can be carried out with for example a vial or syringe, via gentle swirling or rolling between the hands at a slight angle for a short time, such as from 5 to 30 seconds. Thus, the applicant has additionally found that following preparation of the protein solution via the present methods, and prior to either transfer of the solution from a vial to a syringe for delivery to a subject, or delivery to a subject via syringe, or transfer to a suitable container for storage, subjecting the reconstitution vessel to gentle rotation at a slight angle can prove advantageous for the provision of a solution having a consistent concentration throughout. An additional benefit of this post centrifugation step may be inclusion of any minimal amounts of dry protein formulation which were retained on the sides of the vessel during protein transfer i.e. residual powder flecks.

Thus the present invention additionally provides a further reconstitution method as defined hereinbefore which optionally includes the step wherein the resultant protein solution is subject to gentle mixing to remove any residual concentration differences.

Foam includes both full and partial layers of foam around the surface of the reconstituted solution. The reconstituted protein solutions prepared according to the methods of the present invention exhibit minimal foaming. Minimal foaming includes solutions which are substantially foam free. The presence of a few bubbles at the solution surface or within the solution is not considered to constitute persistent foam and is included within the definition of substantially foam free.

Advantageously the method of the present invention provides reconstituted formulations from dry proteins which exhibit minimal foaming and are free of persistent foam. As indicated hereinbefore, the presence of persistent foam in reconstituted solutions can be related to a reduction in protein integrity i.e. denaturation which is associated with protein aggregation and the potential for an immune response. Thus the present methods provide a reduced risk of protein denaturation versus the current methods.

Thus the present invention additionally provides a further reconstitution method as defined hereinbefore which provides reconstituted formulations from dry proteins which exhibit minimal or no foam.

Any suitable dry protein formulation, or combination of dry protein formulations, may be used in the reconstitution method of the present invention including: lyophilized protein powders or cakes; precipitated protein powders or cakes; vacuum dried powders or cakes; air-dried powders or cakes; spray dried powders or cakes; and supercritical fluid dried powders or cakes. Preferred dry formulations for use in the reconstitution methods herein are the amorphous precipitated protein particles prepared according to the method of the present invention as detailed hereinbefore.

Thus the present invention additionally provides methods as described hereinbefore wherein a mixture of dry protein formulations are transferred into a suitable reconstitution vessel, and wherein said mixture of dry protein formulations may be different dry formulations of the same protein or dry formulations of more than one protein.

The present invention additionally provides methods for simultaneous yet independent reconstitution of one or more dry protein formulations, which may be the same or different formulations, in separate reconstitution vessels.

Aqueous diluents suitable for use in the reconstitution method of the present invention include: water for injection (WFI), distilled water, deionised water; sterile water for injection (SWFI); and bacteriostatic water for injection (BWFI) i.e. sterile water with a suitable antimicrobial preservative. The aqueous diluent may additionally comprise one or more buffers, surfactants, salts, stabilizers; or mixtures thereof. Buffers, surfactants, salts, and stabilizers suitable for use in the reconstitution method of the invention can be selected from those well-known in the art. The relative amount of aqueous diluent, including where present buffers, surfactants, salts, or stabilizers or mixtures thereon, will be dependent upon the concentration of the target reconstituted protein solution. Selection of suitable buffers, surfactants, salts and stabilizers for use in any particular aqueous diluent will be dependent upon the particular dry protein formulation to be reconstituted.

The applicants have found that when preparing very highly concentrated reconstituted solutions of protein that use of an aqueous diluent comprising an aqueous solution of the same protein as that to be reconstituted is advantageous.

An example of the reconstitution method comprising protein in the aqueous diluent is provided herein at Example 7.

Thus the present invention additionally provides a method for the reconstitution of dry amorphous precipitated protein formulations comprising:

i) transfer of dry protein formulation into a suitable reconstitution vessel, or preparation of a dry protein formulation within a suitable reconstitution vessel;

ii) addition of a suitable quantity of an aqueous solution of the same protein as used in step (i) into the reconstitution vessel

iii) centrifugation of the reconstitution vessel at a suitable relative centrifugal force for sufficient time to obtain complete or near complete reconstitution of the dry protein formulation into the aqueous diluent and to produce a protein solution that exhibits minimal foaming;

wherein the order of steps (i) and (ii) may be reversed or combined providing for the transfer of diluent to the vessel followed by addition of dry protein, and providing for the transfer of a preformed mixture of dry protein and diluent to the vessel;

and wherein the aqueous protein solution of (ii) may optionally include one or more buffers, surfactants, salts, stabilizers; or mixtures thereof.

The amounts of dry amorphous precipitated protein formulation and aqueous diluent used in accordance with the reconstitution methods of the present invention, as described for steps (i) and (ii), will determine the protein concentration in the resultant reconstituted solution.

The applicant has also found that the reconstitution methods of the invention become increasingly advantageous as the target concentration of the protein solution is increased and the volume of the solid dry protein formulation becomes greater.

High, greater than about 100mg/ml, and very high, from about 140mg/ml to about 350mg/ml, concentrations of protein solutions are especially desirable in the preparation of drug formulations for parenteral administration, such as for example by delivery via a single low volume injection.

In such concentrated and highly concentrated conditions mixing of the solid, dry amorphous precipitated protein formulation, with the added aqueous diluent becomes harder to achieve using conventional reconstitution protocols.

Using the improved reconstitution methods according to the present invention the difficulties associated with conventional protocols are avoided via centrifugal reconstitution. The applicant has found that effective reconstitution is achieved using the improved methods herein even with very high relative amount of solid in the reconstitution vessel. Examples 1 and 3 hereinafter illustrate the advantages of reconstitution of highly concentrated (>200mg/ml) protein solutions via the present methods in comparison to those provided via conventional protocols. Example 7 demonstrates the rapid reconstitution of a dry amorphous precipitated protein formulation to a very high protein concentration (>250mg/ml) via the reconstitution method according to the present invention.

As will be appreciated complete reconstitution, has been achieved when one skilled in the art judges that the dry amorphous precipitated protein particle formulation has been fully reconstituted and no further action is required, in other words it is considered to be optically clear. A fully reconstituted sample will typically be a homogeneous solution or homogeneous dispersion. Near complete reconstitution will arise if one skilled in the art judges that the dry amorphous precipitated protein particle formulation is very close to complete reconstitution. For example, near complete reconstitution may occur if there remains a small amount of material which has not been fully reconstituted, or if the solution is not homogeneous. A small amount of material remaining, typically less than 5% or 1% of the total amount of the dry formulation originally present, may remain attached to the walls of the reconstitution vessel and may be above the level of the solution. Typically it is possible to achieve complete reconstitution of a sample that has reached near complete reconstitution by applying an additional gentle mixing process such as swirling the reconstitution vessel as described herein in order to provide an optically clear solution.

For the avoidance of doubt, complete reconstitution and complete dissolution provide solutions which are considered to be optically clear. Similarly, near complete reconstitution and near complete dissolution provide solutions which one skilled in the art judges to be very close to complete reconstitution or complete dissolution. The terms reconstitution and dissolution as defined herein are interchangeable. Complete reconstitution / dissolution as defined herein will arise if the dry formulation fully dissolves to produce an optically clear solution. This optically clear solution should exhibit a turbidity of less than 10 NTU when diluted to a protein concentration of 10 mg/ml. Preferably complete reconstitution / dissolution will be obtained either on initial treatment or after gentle swirling as indicated herein.

Thus the reconstitution methods of the present invention can advantageously be used for the rapid reconstitution of dry amorphous precipitated protein formulations into protein solutions at high concentrations of greater than about 100mg/ml, and particularly at very high concentrations in the range of from about 140mg/ml to about 350mg/ml, preferably from about 190mg/ml to about 350mg/ml and especially from about 200 mg/ml to about 300mg/ml. In particular the methods of the present invention can provide at least an about 25% reduction in reconstitution time for very high protein concentration solutions when compared to still or continuous or intermittent hand swirling protocols. Preferably the methods of the present invention can provide at least about 50% or at least about 90% reduction in reconstitution time for very high protein concentration solutions when compared to still, continuous or intermittent hand swirling protocols. Thus the present invention provides methods for a reduction of from at least about 25% to at least about 90%, more preferably from at about 50% to about 90% reduction in reconstitution times for very highly concentrated protein solutions when compared to still or continuous or intermittent hand swirling protocols.

According to the present invention there are provided rapidly reconstituted, substantially foam free solutions of highly concentrated, or very highly concentrated protein when prepared from corresponding dry amorphous precipitated protein formulations in accordance with the reconstitution methods herein.

The relative centrifugal force, applied to the reconstitution vessel in the centrifuge, needs to be sufficient to cause the dry amorphous precipitated protein formulation to become partially or completely immersed in the aqueous diluent and preferably to accelerate the reconstitution process. The relative centrifugal force (RCF) expressed in units of gravity (times gravity or xg), increases as the rotor speed of the centrifuge increases. Many microcentrifuges only have settings for speed (revolutions per minute, RPM), not relative centrifugal force. Consequently, a formula for conversion is required to ensure that the appropriate setting is used in an experiment. The relationship between RPM and RCF is as follows:

$$RCF = (1.118 \times 10^{-5}) \, R \, S^2$$

Where RCF is the relative centrifugal force, R is the radius of the rotor in centimetres (cm), and S is the speed of the centrifuge in revolutions per minute (RPM). Values of RCF are commonly quoted in units of times gravity (xg). As an example, centrifugation of a sample at 5,000 RPM in a microcentrifuge that has a rotor with a radius of 7 cm will deliver a relative centrifugal force of 1,957 xg.

On a small scale suitable relative gravitational forces can be conveniently achieved by centrifuging the reconstitution vessel in a laboratory centrifuge such as a bench-top centrifuge, or microcentrifuge. For larger scale processes for concentrating protein an industrial centrifuge, such as a decanter centrifuge, may be used. Reconstitution of concentrated protein solutions in accordance with the method of the present invention can be delivered via centrifugation at a relative centrifugal force of from about 10 xg to about 10,000 xg, and preferably at from about 250 xg to about 5000 xg.

If the reconstitution vessel is an unusual shape such as a pre-filled syringe a suitable insert may be required to hold it securely within the rotor. The need for and preparation of such a suitable insert is considered to be within the remit of the skilled formulator. Alternatively a bespoke device could be used to apply a suitable centripetal acceleration to the reconstitution vessel.

As the centrifugal forces required for application of the present methods are moderate many types of reconstitution vessels are suitable for use including vials, bottles, centrifuge tubes and syringes. Preferred reconstitution vessels are those compatible with fill-finish protocols for dry protein products such as vials or syringes such as pre-filled syringes single chamber or dual chamber syringes.

For preparation of a sterile product it is preferable for the sterile aqueous diluent to be added aseptically to a sterile dry protein formulation that is sealed within the sterile reconstitution vessel.

The ability to use a simple automated process for reconstituting therapeutic dry amorphous precipitated protein formulations for high concentration delivery without foaming / substantially foam free/ with minimal foaming provides a major potential benefit for health care practitioners or self-administering patients with chronic conditions and is a further aspect of this invention. Rather than having to spend time intermittently swirling a vial or syringe until reconstitution is complete, a process that is very hard to carry out reproducibly and which often produces some potentially detrimental foaming, the patient or practitioner is able to simply add the aqueous diluent to the dry protein formulation and place the vial or syringe into a centrifuge for a pre-determined time and at a particular temperature, according to the provided product reconstitution guidelines. A warning signal can be used to alert, for example, the nurse, patient or doctor that reconstitution is complete and on removal from the centrifuge the high concentration protein solution is ready to be administered immediately by, for example, subcutaneous injection. Preferably the centrifuge is provided with the supply of drug and is pre-programmed to carry out the reconstitution using the most appropriate conditions. Alternately a multi-product centrifuge can be used with specific centrifuge reconstitution protocols programmed in for each product. It is also anticipated that the diluent may also be added to the dry protein formulation by an automated device and that the final gentle mixing step may also be automated. This would allow the complete reconstitution process to be carried out without manual intervention.

The reconstitution method of the invention can advantageously be used for the reconstitution of highly temperature sensitive molecules. Thus, using a temperature controllable centrifuge the reconstitution may advantageously be carried out at a low temperature such as 4°C. This would be very difficult to achieve using hand swirling protocols.

The dry amorphous precipitated protein particle formulations prepared by the above process can typically be reconstituted rapidly to high protein concentration to provide clear solutions using the reconstitution methods of the present invention.

According to a further aspect still the present invention additionally provides amorphous dry precipitated protein particles suitable for reconstitution into highly concentrated or very highly concentrated protein solutions according to the reconstitution methods of the present invention and wherein the amorphous dry precipitated protein particles are prepared according to either the preferred method for preparing dry amorphous precipitated protein formulations for reconstitution into highly concentrated protein solution suitable for subcutaneous administration as described herein or via the method of the preparation of amorphous dry precipitated protein particles suitable for reconstitution into highly concentrated protein solutions as described substantially hereinbefore.

Examples

The following Examples are illustrative of specific embodiments of the method and formulations for use in the present invention and are not intended to be limiting thereupon.

Example 1

Preparation of coprecipitated dry protein formulations for reconstitution to high protein concentration

The proteins coprecipitated were PmAb1, a humanised monoclonal antibody, IgG2Δ an isotype, and PmAb2 a human monoclonal antibody, IgG2 isotype.

The dry powder protein formulations for use in this example, and others, were prepared from an aqueous solution comprising the protein, sucrose, and other additives such as buffers, stabilisers and surfactants as detailed in Table 1. The aqueous solution was subsequently coprecipitated by combination and prompt mixing with a 20 fold greater volume of solvent, isobutanol (2-methyl-1-propanol). The aqueous solution and solvent were at the same temperature of about 20°C.

Either batch mixing or continuous flow mixing processes may be used to combine the aqueous solution with the solvent. The following is representative of the batch process used in the preparation of the coprecipitated dry protein formulations in this Example. Into a 125 ml Duran flask was placed the required volume of solvent, typically 60 ml. To this was added a 45 mm magnetic stirring bar. The flask, containing the solvent and the stirrer bar was placed on top of a magnetic stirrer and stirred at 1500 rpm. The aqueous solution, typically 3 ml, containing the protein coprecipitant/buffers/surfactant was added to the solvent by steady drop-wise addition into the middle of the vortex. After completing the addition of the aqueous solution the semi-formed suspension was mixed for from about 1 to about 5 minutes to ensure that the precipitation processes were complete.

The corresponding continuous flow precipitation process would combine a pumped stream of the aqueous mixture with a 20 fold greater pumped stream of the water miscible organic solvent (e.g. isobutanol) using suitable in- line mixers as has already been described in the art for preparing protein-coated microcrystals.

The resultant solvent suspension of precipitated protein-comprising, particles were isolated from the bulk solvent using a membrane filter or a filter tube. The filtered particles were via air dried or vacuum-dried at room temperature or else dried by extraction with supercritical fluid carbon dioxide. To dry the filtered particles, isolated via filter tube, via extraction, the filter tube was transferred to a Thar supercritical fluid extraction rig and supercritical $CO_2$ was typically flowed through the powder at 24 g/min $CO_2$, 100 bar, 45 °C, 90 minutes, followed by depressurisation at a rate of 0.5 bar/10 seconds.

Following drying the powders were transferred from the membrane filter and filter tubes to vials for storage. If required the whole filter cake may be carefully transferred to the vial without breaking it

Table 1 summarises the composition of aqueous solutions used to coprecipitate representative dry powder protein formulations. The theoretical percentage loading is calculated by assuming all of the components in the aqueous mixture precipitate except for the surfactants, polysorbate 20 (PS20) and Tween80 which dissolve in the solvent. The measured protein loading for this and subsequent examples was determined by dissolving a known mass of powder into aqueous solution and measuring the protein concentration by using the measured UV absorbance and the molar extinction constant for the protein.

Table 1

| Formulation identifier | Composition of aqueous solution for precipitation (F= filtered air dried, SC = filtered supercritical fluid extraction) | Percentage loading of protein in dry protein formulation (%w/w) | Description of dry protein formulation |
|---|---|---|---|
| XB_RECON_18_3 | 78.8 mg/mL PmAb1<br>652.5 mg/mL sucrose<br>1.0 mg/mL histidine<br>0.13 mg/mL PS20 (F) | 10.75 (theoretical) | Very sticky, lumpy material stuck to filter membrane |
| XB_RECON_18_2 | 105 mg/mL PmAb1<br>270 mg/mL sucrose<br>1.39 mg/mL histidine<br>0.18 mg/mL PS20 (F) | 27.89 (theoretical) | Very sticky, like chewing gum stuck to the filter, but spread evenly over membrane. |
| XB_RECON_18_1 | 108.75 mg/ml PmAb1<br>167.5 mg/ml sucrose<br>1.40 mg/ml histidine<br>0.18 mg/ml PS20 (F) | 39.14 (theoretical)<br>30.03 (measured) | Waxy material; large particles which stick together. Peeled from filter paper |
| XBCENRECC1_82 | 116.4 mg/ml PmAb1<br>80 mg/mL sucrose<br>1.552 mg/m histidine<br>0.2 mg/ml PS20 pH 5.5 | 58.74 (theoretical)<br>62.8 (measured) | Dry Powder - easy to handle and weigh |
| XBCENREC1_84 | 120 mg/ml PmAb1<br>80 mg/ml sucrose<br>10 mM histidine/histidine:HCl buffer pH 5.5 (SC) | 54.3 (measured) | Dry Powder - easy to handle and weigh |
| XBCENREC1_86_1+2 | 114.2 mg/ml protein PmAb1 70 mg/ml sucrose, 5.5 mg/ml lactobionic acid, 2.7 mg/ml arginine, 0.2 mg/ml PS20, pH 5.5 (SC) | 59.3 (theoretical)<br>58.3 (measured) | Dry Powder - easy to handle and weigh |
| XBCENREC1_86_3+4 | 113.2 mg/ml PmAb1, 70 mg/ml sucrose, 2.3 mg/ml glutamic acid, 2.7 mg/ml arginine, 0.2 mg/ml PS20, pH 5.5 (SC) | 60 (theoretical)<br>59.7 (measured) | Dry Powder - easy to handle and weigh |
| XBCENREC1_86_5+6 | 130.8mg/ml PmAb1, 70 mg/ml sucrose,22.4 mg/ml lactobionic acid, 10.9 mg/ml arginine, 0.2 mg/ml PS20, pH 5.5 (SC) | 55.8 (theoretical)<br>53.1 (measured) | Dry Powder - easy to handle and weigh |
| XBCENREC1_86_7+8 | 114.8 mg/ml PmAb1, 70 mg/ml sucrose, 4.1 mg/ml glutamic acid, 4.9 mg/ml arginine, 0.2 mg/ml PS20, pH 5.5 (SC) | 57.59 (theoretical)<br>59.2 (measured) | Dry Powder - easy to handle and weigh |
| XB_RECON_058_01 | 122.3 mg/ml PmAb1 70 mg/ml sucrose, 5.5 mg/ml lactobionic acid, 2.7 mg/ml arginine, 0.2 mg/ml PS20, pH 5.5 (SC) | 60.5 (theoretical)<br>58.7 (measured) | Dry Powder - easy to handle and weigh |
| XB_RECON_058_02 | 126.68 mg/ml PmAb1, 70 mg/ml sucrose, 2.3 mg/ml glutamic acid, 2.7 mg/ml arginine, 0.2 mg/ml PS20, pH 5.5 (SC) | 61.5 (theoretical)<br>60.7 (measured) | Dry Powder - easy to handle and weigh |

(continued)

| Formulation identifier | Composition of aqueous solution for precipitation (F= filtered air dried, SC = filtered supercritical fluid extraction) | Percentage loading of protein in dry protein formulation (%w/w) | Description of dry protein formulation |
|---|---|---|---|
| XB_RECON_058_03 | 120.6 mg/ml PmAb1, 70 mg/ml sucrose, 4.1 mg/ml glutamic acid, 4.9 mg/ml arginine, 0.2 mg/ml PS20, pH 5.5 (SC) | 60.2 (theoretical) 59.4(measured) | Dry Powder - easy to handle and weigh |
| XB_RECON_32_03 | 110.8 mg/mL PmAb2 80 mg/mL sucrose 20 mM histidine 0.2 mg/mL Tween 80 (SC) | 57.08 (theoretical) 55.92 (measured) | Dry Powder - easy to handle and weigh |

**Discussion of results**

The preparation of dry protein formulations via coprecipitation using aqueous solutions comprising a mass concentration of protein lower than the sucrose mass concentration was found to result in sticky gels or cohesive waxy powders (Formulations XB_RECON_18_1, XB_RECON_18_2, and XB_RECON_18_3). Furthermore formulation XB_RECON_18_1 which had a theoretical protein loadings of ~39 %w/w gave a measured protein loading of ~30% showing a quarter of the mass of protein had been lost during the process. Such a loss would not be sustainable in a manufacturing process. The theoretical protein loadings of these formulations were in the range 1-40 %w/w which has previously been reported to be the preferred range for preparing protein coated microcrystals, as disclosed at page 24 of WO 2004/062560A2. These poor results support the applicant's view that, based on the teachings of the art, sucrose would be unlikely considered suitable as a coprecipitant for preparing dry powder formulations of proteins. Indeed prior to this invention sucrose has not been considered to be a preferred excipient for the coprecipitation of protein particles using water miscible solvents possibly because the observed behavior (described hereinbefore) indicates that sucrose has an inherently slow rate of precipitation and/or crystallization in the solvent.

As shown in Table 2, the inventors have now surprisingly found that if the mass concentration of protein in the aqueous solution is increased so it is higher than the sucrose mass concentration, coprecipitation of sucrose and protein can be successfully carried out and dry powders can be straightforwardly isolated in good yields. The measured protein loadings of the remaining precipitated protein particle formulations shown in Table 1 are in the range of from 54 to 65 %w/w and are similar to the theoretical protein loadings. This surprising change in behavior for sucrose has not been reported previously, possibly because to observe it requires the protein concentration to be much higher than would commonly be used in trial experiments. The close correlation of the measured and theoretical loading shows that the mixture of protein, sucrose and excipients have coprecipitated together to form particles. Significantly, SEM images and powder X-ray diffraction (PSRD) show that these particles are completely or very substantially amorphous amorphous. PXRD traces show a featureless broad peak (halo) while SEM shows the particles are spherical or donut shaped with a diameter of 100 microns or less. They are therefore clearly distinct from previously disclosed protein-coated microcrystals which contain a crystalline core and are typically non-spherical, forming rods or plates. Nevertheless as demonstrated below in Example 2 the protein contained within these high loaded particles still shows very high retention of integrity and minimal aggregation even though it has been exposed to a 95% water-miscible protic solvent. Importantly these powders containing amorphous dry precipitated particles comprising protein, sucrose and excipients are found to be highly suitable for reconstitution to high protein concentrations.

This novel method for preparing high loaded dry amorphous precipitated protein particles comprising sucrose provides a surprising and advantageous route for formulating proteins. In particular, it may be used as an attractive method for preparing dry formulations which can be reconstituted to form an aqueous solution suitable for subcutaneous administration of proteins at high concentration, such as greater than 120 mg/ml protein or greater than 200 mg/ml as shown in Example 2.

Example 2

Reconstitution of dry amorphous precipitated protein formulations to high protein concentration

Whether the amorphous precipitated protein particles comprising sucrose could be reconstituted to high concentration and whether in so-doing they would retain protein integrity was investigated. Precipitated particles were prepared as described in Example 1. Aqueous solutions of the various compositions detailed in Table 1 were combined rapidly with a 20 fold larger volume of isobutanol at a temperature of 20 °C. Each of the resultant miscible solvent mixtures contained a suspension of amorphous precipitated particles which were subsequently dried by extracting the solvent mixture with supercritical carbon dioxide to furnish dry amorphous precipitated protein formulations of the invention. Each of these formulations was then reconstituted to a target concentration of about 200 mg/ml and the reconstitution time, turbidity, osmolality and monomer content were measured.

Reconstitution was achieved using a swirling reconstitution protocol, in which the diluent (deionized water) was added, and after waiting for 30 seconds the vial was swirled by hand 10 times keeping the base of the vial on the surface of the bench. Every 5 minutes the vial was hand swirled 10 more times. Osmolality was measured by freezing point depression using a Gonotec Osmomat 030, and turbidity was measured using a Hach Lange 2100 AN Turbidimeter, both measurements being carried out according to standard methods known in the art. For monomer content and turbidity measurements the solution was diluted down to a protein concentration of 10 mg/ml. The monomer content was measured using size exclusion chromatography (SEC) according to methods known in the art. The results are shown in Table 2.

Table 2

| Sample | Protein concentration in reconstituted solution (mg/ml) | Turbidity (NTU) | Osmolality (mOsmol/kg) | Monomer Content by SEC (%) | Reconstitution time by swirling (min) |
|---|---|---|---|---|---|
| XBCENREC1_86_1+2_A | 203 | 6 | 568 | 98.1 | > 72 minutes (Left overnight) |
| XBCENREC1_86_3+4_A | 214 | 3 | 560 | 98.1 | > 72 minutes (Left overnight) |
| XBCENREC1_86_5+6_A | 203 | 4 | 727 | 98.6 | > 72 minutes (Left overnight) |
| XBCENREC1_86_7+8_A | 214 | 4 | 507 | 97.9 | > 72 minutes (Left overnight) |
| XB_RECON_32_03 | 218 | 5 | 755 | 97.2 | 90 minutes |

It can be seen that at the high target concentration of around 200 mg/ml PmAb1 formulations took greater than 72 minutes to reconstitute the amorphous precipitated protein formulations using a standard swirling method. The samples were therefore left overnight to complete the process. More promisingly it can be seen that, once reconstituted, the monomer content observed was good given the initial reconstitution has been to high concentration with all samples in the range of from about 97.9% to about 98.6%. Similarly the turbidity observed was low showing that there was no evidence indicating the formation of visible particles. Finally, with the exception of one sample, the observed osmolality was lower than about 600mOsmol/kg, which is low enough for the solutions to be acceptable for subcutaneous injection.

These results demonstrate that for all dry formulation containing amorphous precipitated particle comprising protein, sucrose, arginine and either glutamic acid or lactobionic acid, the PmAb1 (protein) has retained good integrity during the whole cycle of precipitation, supercritical-fluid drying and reconstitution to high concentration. Similar results were obtained for the PmAb2 formulation. Whilst the solutions so-produced are indeed likely to be suitable for subcutaneous administration their reconstitution times are potentially too long for many commercial products.

Example 3

Syringability and osmolality of concentrated protein solutions prepared from reconstituted precipitated particles.

The suitability of the amorphous precipitated protein particles prepared by the method of the present invention for subcutaneous administration was determined by measurement of their glide force and osmolality. Precipitated particles were prepared as described in Example 1. Aqueous solutions of the composition shown in Table 1 were combined rapidly with a 20 fold larger volume of solvent (isobutanol) at a temperature of 20°C. The resultant miscible solvent mixture contained a suspension of amorphous precipitated particles which were dried by extracting the solvent mixture with supercritical carbon dioxide. For each of the compositions the measured protein loading (MPL) (see Example 1) was found to correspond closely to the theoretical protein loading (TPL) showing that the composition of the precipitated particles was very similar to that of the solids present in the original aqueous solution. Thus, each particle comprises a mixture of protein, sucrose, arginine and glutamic acid or lactobionic acid.

A Stable Micro Systems Texture Analyser (TA-XT$^{PLUS}$) was used to assess the syringability of solutions formed on reconstitution of the amorphous dry protein formulations produced by the coprecipitation process. The Texture Analyser directly measures the force required to expel a particular protein solution from a syringe through a hyperdermic needle, the so-called glide force. As well as the particular protein solution the glide force measured will depend on the syringe used, the gauge of the needle, the temperature and the speed at which the plunger is depressed.

Together these parameters determine the speed at which the solution can be pushed through the needle. By fixing these parameters, it is possible to compare the syringability of different solutions when expelled through the same needle as the same rate. In addition, by measuring the glide force obtained for standard solutions of known viscosity under the same fixed conditions, it is also possible to make comparisons with the viscosity data reported for other concentrated protein solutions.

The conditions used for making the glide force measurements in this example were as follows: Becton Dickinson S.A. Plastipak 1mL syringe (309602); Becton Dickinson 27 G needle ½" (300635). The Texture Analyser conditions used for making the syringability measurements in this example were as follows: 345 mm/min displacement speed; 17.5mm displacement distance. Using these parameters a volume of 350 µl of solution was expelled from the syringe in ~3.6 seconds. The solution was therefore passed through the 27 G needle at about 97 µl/s and the average force measured between 10-15mm was taken as the glide force. The experiments were run at a temperature of about 20°C.

The glide forces measured following reconstitution of various dry powder formulations of amorphous precipitated protein particles as described in Example 2 to make high concentration protein solutions are shown in Table 3. The protein concentration and osmolality of the samples were measured by methods known in the art. Osmolality of the sample was measured by a freezing point depression method using a Gonotec Osmomat 030 and standard measurement techniques as are known in the art.

The results obtained demonstrate that solutions can be produced by reconstitution of amorphous precipitated particles which have a protein concentration of greater than about 200 mg/ml and an osmolality of less than about 600 mOsmol/kg.

The results also show that concentrated protein solutions prepared and reconstituted from dry amorphous precipitated particles according to the invention show good syringability with a glide force of significantly less than 15N when passed through a ½ inch 27 G needle at a flow rate of 100µl/s at 20°C.

Table 3

| Sample | Protein loading (%w/w) | Protein concentration (mg/ml) | Osmolality (mOsmol/kg) | Measured Glide force (Newtons) |
|---|---|---|---|---|
| XB_RECON_ 058_01 | 58.73 | 218 | 583 | 10.5 |
| XB_RECON_ 058_02 | 60.69 | 210 | 527 | 9.0 |
| XB_RECON_ 058_03 | 59.37 | 219 | 598 | 5.8 |

Example 4

Determination of whether the reconstitution of dry amorphous precipitated protein formulations can be accelerated using a centrifugation method

Samples of the dry amorphous precipitated protein formulation XBCENREC1_82 (Table 1) prepared and dried by supercritical fluid extraction according to Example 1, were reconstituted in accordance with either conventional swirling methods known in the art or a novel centrifugation method.

Experimental Methods

The precipitated dry formulation XBCENREC1_82, comprising 62.8%w/w of protein was tested in 5 concurrent reconstitution experiments carried out at an ambient temperature of 22°C in identical 6ml glass lyophilisation vials. In each experiment approximately the same measured mass (318 mg) of dry powder and approximately the same volume (750 µl) of deionized water (>15 MΩ from Millipore Elix 10 system) were used.

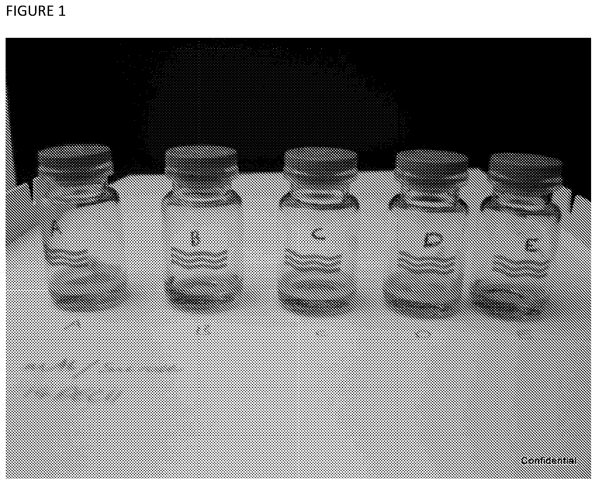

In vials XB1A, XB1B and XB1C the water was added onto (above) the dry powder, whilst in vials XB1D and XB1E the water was injected underneath the dry powder using a syringe with a 27G needle. Vials XB1A and XB1D were then left undisturbed during the reconstitution process and photographs were taken periodically. Vials XB1B and XB1E were carefully placed into an ALC Refrigerated Centrifuge PK130R centrifuge (T535 4-fold swing-out rotor with P510 cups and 4 piece Falcon tube adaptor) and centrifuged at 2500 rpm at a temperature of 22 °C. To cushion the base of the vial during centrifuging a plug of crumpled paper was placed in the base of the adaptor. The relative centrifugal force applied to the vial was estimated to be about 1000 xg. After 10 minutes the centrifuge was stopped and vials XB1B and XB1E were removed and a photograph taken. Vial XB1C was left undisturbed for 30 seconds after adding the diluent and then swirled gently by hand 10 times. This hand swirling process was repeated every 5 minutes until full reconstitution was achieved. A photograph was taken of all the vials after 10 minutes as shown in Figure 1.

Figure 1 illustrates 5 vials A to E containing the reconstituted solutions XB1A to XB1E respectively.

Discussion of Results

After 10 minutes as illustrated in Figure 1, the vials XB1B and XB1E treated in accordance with the method of the present invention contained an optically clear foam-free concentrated protein solution. This confirmed that there was no significant difference in the rapidity of reconstitution or provision of foam free solutions when using the method of the present invention where the diluent was added to the protein or the protein was added to the diluent. As also illustrated in Figure 1, the undisturbed vials XB1A and XB1D were found to still contain a noticeable mass of undissolved powder demonstrating that the reconstitution process remained far from complete at the 10 minute point using the undisturbed protocol. Figure 1 illustrates that although the hand swirled vial XB1C had much less powder remaining than in the undisturbed vials XB1A and XB1D the reconstitution process remained far from incomplete at 10 minutes and also that the repeated hand swirling protocol had generated a significant layer of foam.

These results demonstrate that the reconstitution method of the invention provides significant advantages in terms of reconstitution time versus either the still or hand swirling protocols in current use.

An aliquot was taken from vials XB1A and XB1B after leaving overnight and the protein concentration was measured. Size-exclusion chromatography (SEC) measurements of the relative compositions of the aliquots were used to determine the effects on protein integrity of the reconstitution method of the invention versus the still (undisturbed) protocol in current use. No discernible difference was observed between samples prepared by either method.

These results confirm that reconstitution of a coprecipitated dry amorphous protein formulation with an aqueous diluent according to the method of the present invention surprisingly and advantageously not only provides for shorter reconstitution times to prepare a high protein concentration solution than either the undisturbed or hand swirling protocols, but also produces a clear foam free solution which gives identical protein integrity to simply leaving the solution undisturbed.

Example 5

The four dry protein formulations XBCENREC1_86_1+2 to XBCENREC1_86_7+8 (Table 2) were prepared and dried by supercritical fluid extraction according to the method described in Example 2. A comparison was made of the reconstitution times required to prepare >200 mg/ml solutions from these dry powders by either a hand swirling reconstitution protocol or a centrifuge reconstitution protocol. Both protocols used 6 ml clear glass vials (Chromacol, crimp top, 6-CV) as the reconstitution vessels. For the centrifuge reconstitution process the aqueous diluent (deionized water) was added to the dry powder in the vial which was then carefully placed into an ALC Refrigerated Centrifuge PK130R centrifuge (T535 4-fold swing-out rotor with P510 cups and 4 piece Falcon tube adaptor) and centrifuged at 2500 rpm at a temperature of 22°C. To cushion the base of the vial during centrifuging a plug of crumpled paper was placed in the base of the adaptor. After 5, 10 and 15 min the centrifuge was stopped the vials carefully removed and a photograph taken. The reconstitution time from addition of diluent, to the formation of a completely clear solution containing no visible particles was recorded. The protein concentration, osmolality, turbidity and monomer content were measured for each reconstituted solution according to the techniques detailed hereinbefore and are summarized in Table 4.

Table 4

| Sample | Reconstitution Protocol | Mass of dry protein formulation (mg), volume of deionised water added (ml) | Description after 10 minutes | Reconstitution Time (minutes) | Protein concentration in reconstituted solution (mg/ml) |
|---|---|---|---|---|---|
| XBCENREC1_86_1+2_A | Swirling | 343, 0.75 | Large clump submerged. 60% powder dissolved. Very little foaming | > 72 Left overnight. | 203 |
| XBCENREC1_86_1+2_B | Centrifuge | 343, 0.75 | Fully reconstituted, no bubbles or foam | 10 | 210 |
| XBCENREC1_86_3+4_A | Swirling | 335, 0.75 | Large clump submerged in diluent. 75% Very little foaming. | >72 Left overnight for analysis | 214 |
| XBCENREC1_86_3+4_B | Centrifuge | 335, 0.75 | No bubbles. < 5% remaining. | 15 | 210 |
| XBCENREC1_5+6_A | 86 Swirling | 377, 0.75 | Significant foaming, 90% of powder dissolved. | >72 Left overnight for analysis | 203 |
| XBCENREC1_86_5+6_B | Centrifuge | 377, 0.75 | Fully reconstituted, no bubbles or foam | 10 | 202 |
| XBCENREC1_86_7+8_A | Swirling | 347, 0.75 | Foaming. Couple of smears. Large plug remaining- 90% dissolved. | >72 Left overnight for analysis | 214 |
| XBCENREC1_86_7+8_B | Centrifuge | 347, 0.75 | Fully reconstituted, no bubbles or foam | 10 | 210 |

These results demonstrate that the centrifugation method of the invention is able to significantly reduce the time taken to reconstitute 4 different formulations of dry amorphous precipitated particles comprising protein, sucrose and precipitation stabilizing additives to very high concentrations (>200mg/ml). Centrifugation has also been demonstrated to prevent the formation of bubbles or foam during reconstitution.

Example 6

Reconstitution of dry powders and cakes of amorphous precipitated proteins using centrifugation

A comparison was made of the time taken to reconstitute protein solutions of formulation XBCENREC1_84 (Table 1), where said solutions were prepared in accordance with the methods of Example 2, by the swirling protocol and the centrifuge reconstitution protocol for a similar mass of dry powder and with a similar mass of dry powder compressed to form a free-standing dry cake. The reconstitution procedures used were the same as Example 3. Following dilution of the reconstituted solution to a protein concentration of 10 mg/ml the turbidity and monomer content were measured by standard methods known in the art. The results are shown in Table 5. The protein concentration, turbidity and monomer content of the samples were measured by methods known in the art. The monomer content was measured by size exclusion chromatography following dilution of the sample to a protein concentration of 10 mg/ml. The turbidity was also measured at a protein concentration of 10 mg/ml.

The monomer content of the protein in the aqueous solution of PmAb1 used for the precipitation was 98.8 %.

Table 5

| Sample | Sample form | Recon method | Reconstitution time (mins) | Measured Protein Concentration (mg/mL) | Turbidity (NTU) | Monomer content (%) |
|---|---|---|---|---|---|---|
| XBCENRE_ 84_1_A | Dry powder | Swirling | 75 (still foam at the periphery) | 190 | 2.31 | 98.28 |
| XBCENRE_ 84_1_B | Dry powder | centrifuge | 15 | 196 | 1.96 | 98.38 |
| XBCENRE_ 84_1_E | Dry Cake | centrifuge | 20 | 200 | 3.15 | 98.37 |

These results show the centrifuge reconstitution method can be advantageously used with dry protein formulations prepared either as a dry powder or as a dry cake.

Example 7

Reconstituting a dry amorphous precipitated protein formulation into an aqueous diluent containing dissolved protein

Two vials, each containing 750 μ! of a 120 mg/ml aqueous solution of PmAb1 were prepared, and into each was added 293 mg of the dry powder formulation XBCENREC1_84 prepared as described in Example 2. After 30 seconds one of the vials was swirled by hand 10 times keeping the base of the vial on the benchtop. The swirling was repeated every 5 minutes until the powder was reconstituted. The second vial was placed into an ALC Refrigerated Centrifuge PK130R centrifuge (T535 4-fold swing-out rotor with T516 cups and 4 piece Falcon tube adaptor) and centrifuged at 2500 rpm at a temperature of 22°C. Each vial was assessed visually every 5 minutes until reconstitution was complete. A photograph of each vial was taken after 10 and 15 minutes. The protein concentration, turbidity and monomer content of the samples were measured. The monomer content was measured by size exclusion chromatography (SEC) following dilution of the sample to a protein concentration of 10 mg/ml. Turbidity was also measured following dilution of the sample to 10 mg/ml. The monomer content of PmAb1 in the aqueous solution used to carry out the precipitation was 98.8%. The results are provided in Table 6.

Table 6

| Sample | Reconstitution Method | Reconstitution time | Measured Protein Concentration (mg/mL) | Turbidity (NTU) | Monomer content (%) |
|---|---|---|---|---|---|
| XBCENREC1_84_1 | Swirling | 70 minutes - little bit of foaming | 278 | 3 | 98 |
| XBCENREC1_84_2 | Centrifuge | 20 minutes - no foaming - no bubbles | 265 | 6 | 98.5 |

17

This experiment demonstrates that centrifuge assisted reconstitution of a dry amorphous precipitated protein formulation into a solution of the same protein can be used to produce foam-free very high protein concentration solutions in less than 30 minutes.

Example 8

Precipitation via continuous flow and drying of amorphous dry particles comprising protein, sucrose, arginine and lactobionic acid

An aqueous solution was prepared containing 120 mg/ml PmAb1, 70 mg/ml sucrose, 5.5 mg/ml lactobionic acid, 2.7 mg/ml arginine and 0.2mg/ml Polysorbate 20.

Using a continuous flow coprecipitation system, comprising of two Watson Marlow 520 DU pumps fitted with a 505L low pulse head for the aqueous line and a 520REH 4-7bar; 60-100psi head for the solvent line connected with silicone tubing of 3.2 mm internal diameter plus a plastic cross piece mixer from Kartell, a bulk batch of precipitated particles were produced by continuous flow precipitation. The aqueous protein solution was pumped at a flow rate of 50 mL/min whilst the solvent (isobutanol) was pumped at a flow rate of 950 ml/min, giving a total flow rate of 1000 mL/min. Approximately 250 mL of suspension was produced in the 15 second production run. After initial precipitation, the resultant suspension was mixed for 1 minute with a magnetic stirrer unit to ensure that the precipitation process was complete. Thereafter, 63 mL aliquots of suspension were filtered over a 47 mm Millipore Durapore PVDF membrane (0.45 $\mu$m pore size) and transferred to a 12 mL / 1.0$\mu$m filter extraction tubes for SCFE drying in a 6 tube drying run (2 additional tubes were included as blanks to ensure uniform drying across the SCFE vessel). The SCFE drying conditions were as follows: $CO_2$ flow rate 24 g/min; temperature 45°C; pressure = 100 bar; depressurization rate = 0.5bar/ 10 seconds.

Four batches of powder were produced and blended after drying and a combined MPL of 61.6 %w/w was recorded. 324.8 mg of powder (equivalent to 200 mg mAb) was reconstituted using 750 uL of diluent. The sample was spun for 30 seconds at 2500 rpm axially, and then spun for 9.5 minutes at 4000 rpm (2683 RCF) in the conventional revolving axis.

The reconstitution time was measured to be 10 minutes, the measured concentration was 232 mg/mL, the measured osmolality was 591 mOsm/kg at 200 mg/ml, and the monomer content was 98.15%. The monomer contact of the stock material was measured to be 98.87%.

These results demonstrate that the inventive combination of a continuous method for preparing dry amorphous precipitated particles comprising protein, sucrose and protective additive and a method for rapidly reconstitution said particles to very high concentration can be used to provide solutions suitable for parenteral delivery of proteins at very high concentrations.

**Claims**

**1.** A method for the preparation of amorphous dry precipitated particles that comprise protein, sucrose, arginine and glutamic acid or lactobionic acid comprising:

(i) preparation of an aqueous solution for precipitation comprising:

(a) a protein at a mass concentration of greater than 50 mg/ml;
(b) sucrose at a mass concentration between 30 % to 95 % of the mass concentration of the protein;
(c) arginine at a molar concentration of less than 50 mM;
(d) glutamic acid or lactobionic acid at a molar concentration of less than 50 mM;
(e) optionally a surfactant;

(ii) combining the aqueous solution for precipitation with at least a 12 fold volume of one or more protic organic solvents at a temperature in the range of from 15°C to 50°C to produce a miscible solvent mixture and suspension of amorphous precipitated particles which each comprise protein, sucrose, arginine and glutamic acid or lactobionic acid;
(iii) concentrating the suspension and/or removing the remaining solvent to isolate the dry amorphous precipitated particles comprising protein; and

wherein said particles can be reconstituted to form an aqueous solution suitable for subcutaneous administration of said protein at a concentration of greater than 140 mg/ml protein.

2. A method according to claim 1 wherein the protic organic solvent is independently selected from isobutanol, isopropanol, 2-methyl-2,4-pentane diol and mixtures thereof.

3. A method according to Claim 2 wherein the protic organic solvent is isobutanol.

4. A method according to any of the preceding claims wherein the glutamic acid or lactobionic acid; or arginine are independently present at a molar concentration of less than 25 mM.

5. A method according to any of the preceding claims wherein the protein is a therapeutic or diagnostic protein.

6. A method according to any of the preceding claims wherein the protein is a monoclonal antibody or conjugate thereof.

7. A method according to any of the preceding claims wherein the dry amorphous precipitated particles comprising protein are provided from the suspension by supercritical fluid extraction or vacuum or air drying.

8. A method according to any of the preceding claims wherein the osmolality of said aqueous solution comprising >140 mg/ml protein formed on reconstitution of the dry amorphous precipitated protein particles, prepared according to any of claims 1 to 7, is less than 1000 mOsmols/kg, less than 800 mOsmols/kg, or less than 600 mOsmol/kg.

9. A method according to any of the preceding claims wherein the glide force measured for said aqueous solution comprising >140 mg/ml protein formed on reconstitution of the dry amorphous precipitated protein particles, prepared according to any of clams Claim 1 to 8, is <15N when the solution is passed through a standard ½ in 27 G syringe needle at 100 $\mu$l/s at a temperature of 20°C.

10. A method according to any of the preceding claims wherein the dry amorphous precipitated protein particles are reconstituted to a concentration of the protein of greater than 180 mg/ml, greater than 220 mg/ml, greater than 260mg/ml, or greater than 300mg/ml.

11. A method according to any of the preceding claims wherein the dry amorphous precipitated protein particles are reconstituted to a concentration of between 120 mg/ml and 350 mg/ml, or between 180 mg/ml and 300 mg/ml.

12. A method according to any of the preceding claims wherein the diluent for reconstitution of the dry amorphous precipitated protein particles is: water for injection (WFI), distilled water, deionised water; sterile water for injection (SWFI); bacteriostatic water for injection (BWFI); and wherein the diluent may comprise one or more buffers, surfactants, salt stabilizers or mixtures thereof.

13. A method according to any of the preceding claims wherein the dry amorphous precipitated protein particles are reconstituted to form an aqueous solution via centrifugal reconstitution.

14. A method according to claim 13 comprising:

i) transfer of dry amorphous precipitated protein particles into a suitable reconstitution vessel, or preparation of dry amorphous precipitated protein particles within a suitable reconstitution vessel;
ii) addition of a suitable quantity of an aqueous diluent to the reconstitution vessel;
iii) centrifugation of the reconstitution vessel at a suitable relative centrifugal force for sufficient time to obtain complete or near complete reconstitution of the dry amorphous precipitated protein particle formulation into the aqueous diluent and to produce a protein solution that exhibits minimal or no foaming.

15. A method according to claim 13 wherein the diluent for reconstitution is a solution of the same protein, or mixture of proteins as used in step (i).

**Patentansprüche**

1. Verfahren zur Herstellung von amorphen trockenen ausgefällten Teilchen, die Protein, Saccharose, Arginin und Glutaminsäure oder Lactobionsäure umfassen, Folgendes umfassend:

(i) Herstellen einer wässrigen Lösung zum Fällen, Folgendes umfassend:

(a) ein Protein mit einer Massenkonzentration von mehr als 50 mg/ml;

(b) Saccharose mit einer Massenkonzentration zwischen 30 % und 95 % der Massenkonzentration des Proteins;

(c) Arginin mit einer molaren Konzentration von weniger als 50 mmol;

(d) Glutaminsäure oder Lactobionsäure mit einer molaren Konzentration von weniger als 50 mmol;

(e) wahlweise ein Tensid;

(ii) Kombinieren der wässrigen Lösung zum Fällen mit mindestens einem 12-fachen Volumen von einem oder mehreren protischen organischen Lösungsmitteln bei einer Temperatur im Bereich von 15 °C bis 50 °C, um eine mischbare Lösungsmittelmischung und eine Suspension von amorphen ausgefällten Teilchen, welche jeweils Protein, Saccharose, Arginin und Glutaminsäure oder Lactobionsäure umfassen, herzustellen;

(iii) Konzentrieren der Suspension und/oder Entfernen des verbleibenden Lösungsmittels, um die trockenen amorphen ausgefällten Protein umfassenden Teilchen zu isolieren; und

wobei die Teilchen rekonstituiert werden können, um eine wässrige Lösung zu bilden, die zur subkutanen Verabreichung des Proteins mit einer Konzentration von mehr als 140 mg/ml Protein geeignet ist.

2. Verfahren nach Anspruch 1, wobei das protische organische Lösungsmittel unabhängig aus Isobutanol, Isopropanol, 2-Methyl-2,4-pentandiol und Mischungen davon ausgewählt ist.

3. Verfahren nach Anspruch 2, wobei das protische organische Lösungsmittel Isobutanol ist.

4. Verfahren nach einem der vorhergehenden Ansprüche, wobei die Glutaminsäure oder Lactobionsäure oder das Arginin unabhängig mit einer molaren Konzentration von weniger als 25 mmol vorhanden ist.

5. Verfahren nach einem der vorhergehenden Ansprüche, wobei das Protein ein therapeutisches oder diagnostisches Protein ist.

6. Verfahren nach einem der vorhergehenden Ansprüche, wobei das Protein ein monoklonaler Antikörper oder ein Konjugat davon ist.

7. Verfahren nach einem der vorhergehenden Ansprüche, wobei die trockenen amorphen ausgefällten Protein umfassenden Teilchen von der Suspension durch superkritische Fluidextraktion oder Vakuum- oder Lufttrocknen bereitgestellt werden.

8. Verfahren nach einem der vorhergehenden Ansprüche, wobei die Osmolalität der wässrigen Lösung, die > 140 mg/ml Protein, das zur Rekonstitution der trockenen amorphen ausgefällten Proteinteilchen gebildet ist und nach einem der Ansprüche 1 bis 7 hergestellt ist, weniger als 1000 mOsmol/kg, weniger als 800 mOsmol/kg oder weniger als 600 mOsmol/kg beträgt.

9. Verfahren nach einem der vorhergehenden Ansprüche, wobei die Gleitkraft, die für die wässrige Lösung gemessen wurde, die > 140 mg/ml Protein, das zur Rekonstitution der trockenen amorphen ausgefällten Proteinteilchen gebildet ist und nach einem der Ansprüche 1 bis 8 hergestellt ist, umfasst, < 15 N beträgt, wenn die Lösung durch eine standardmäßige ½ in 27-G-Spritzennadel bei 100 $\mu$l/s bei einer Temperatur von 20 °C geleitet wird.

10. Verfahren nach einem der vorhergehenden Ansprüche, wobei die trockenen amorphen ausgefällten Proteinteilchen zu einer Konzentration des Proteins von mehr als 180 mg/ml, mehr als 220 mg/ml, mehr als 260 mg/ml oder mehr als 300 mg/ml rekonstituiert werden.

11. Verfahren nach einem der vorhergehenden Ansprüche, wobei die trockenen amorphen ausgefällten Proteinteilchen zu einer Konzentration von zwischen 120 mg/ml und 350 mg/ml oder zwischen 180 mg/ml und 300 mg/ml rekonstituiert werden.

12. Verfahren nach einem der vorhergehenden Ansprüche, wobei das Verdünnungsmittel zur Rekonstitution der trockenen amorphen ausgefällten Proteinteilchen Folgendes ist: Wasser zur Injektion (WFI), destilliertes Wasser, entionisiertes Wasser; steriles Wasser zur Injektion (SWFI); bakteriostatisches Wasser zur Injektion (BWFI); und wobei das Verdünnungsmittel einen oder mehrere Puffer, Tenside, Salzstabilisatoren oder Mischungen davon umfassen kann.

**13.** Verfahren nach einem der vorhergehenden Ansprüche, wobei die trockenen amorphen ausgefällten Proteinteilchen rekonstituiert werden, um eine wässrige Lösung mittels Zentrifugalrekonstitution zu bilden.

**14.** Verfahren nach Anspruch 13, Folgendes umfassend:

(i) Transfer von trockenen amorphen ausgefällten Proteinteilchen in ein geeignetes Rekonstitutionsgefäß oder Herstellen von trockenen amorphen ausgefällten Proteinteilchen innerhalb eines geeigneten Rekonstitutionsgefäßes;
(ii) Hinzufügen einer geeigneten Menge eines wässrigen Verdünnungsmittels in das Rekonstitutionsgefäß;
(iii) Zentrifugieren des Rekonstitutionsgefäßes mit einer geeigneten relativen Zentrifugalkraft für eine ausreichende Zeit, um eine vollständige oder beinahe vollständige Rekonstitution der Formulierung der trockenen amorphen ausgefällten Proteinteilchen im wässrigen Verdünnungsmittel zu erhalten und eine Proteinlösung herzustellen, die minimale oder keine Schaumbildung aufweist.

**15.** Verfahren nach Anspruch 13, wobei das Verdünnungsmittel zur Rekonstitution eine Lösung des gleichen Proteins oder einer wie in Schritt (i) verwendeten Mischung von Proteinen ist.

**Revendications**

**1.** Procédé de préparation de particules précipitées amorphes sèches qui comprennent une protéine, du saccharose, de l'arginine et de l'acide glutamique ou de l'acide lactobionique comprenant :

(i) la préparation d'une solution aqueuse pour précipitation comprenant :

(a) une protéine à une concentration massique supérieure à 50 mg/ml ;
(b) du saccharose à une concentration massique représentant entre 30 % à 95 % de la concentration massique de la protéine ;
(c) de l'arginine à une concentration molaire inférieure à 50 mM ;
(d) de l'acide glutamique ou de l'acide lactobionique à une concentration molaire inférieure à 50 mM ;
(e) éventuellement un tensioactif ;

(ii) la combinaison de la solution aqueuse pour précipitation avec au moins un volume 12 fois supérieur d'un ou plusieurs solvants organiques protiques à une température comprise dans la plage de 15°C à 50°C pour produire un mélange de solvants miscibles et une suspension de particules précipitées amorphes, chacune comprenant une protéine, du saccharose, de l'arginine et de l'acide glutamique ou de l'acide lactobionique ;
(iii) la concentration de la suspension et/ou l'élimination du solvant restant pour isoler les particules précipitées amorphes sèches comprenant une protéine ; et

lesdites particules pouvant être reconstituées pour former une solution aqueuse appropriée à une administration sous-cutanée de ladite protéine à une concentration supérieure à 140 mg/ml de protéine.

**2.** Procédé selon la revendication 1, ledit solvant organique protique étant indépendamment choisi parmi l'isobutanol, l'isopropanol, le 2-méthyl-2,4-pentane-diol et des mélanges de ceux-ci.

**3.** Procédé selon la revendication 2, ledit solvant organique protique étant l'isobutanol.

**4.** Procédé selon l'une quelconque des revendications précédentes, l'acide glutamique ou l'acide lactobionique, ou l'arginine étant indépendamment présent(e) à une concentration molaire inférieure à 25 mM.

**5.** Procédé selon l'une quelconque des revendications précédentes, ladite protéine étant une protéine thérapeutique ou de diagnostic.

**6.** Procédé selon l'une quelconque des revendications précédentes, ladite protéine étant un anticorps monoclonal ou un conjugué de celui-ci.

**7.** Procédé selon l'une quelconque des revendications précédentes, lesdites particules précipitées amorphes sèches comprenant une protéine étant fournies à partir de la suspension par extraction par fluide supercritique ou séchage sous vide ou à l'air.

8. Procédé selon l'une quelconque des revendications précédentes, l'osmolalité de ladite solution aqueuse comprenant > 140 mg/ml de protéine formée lors de la reconstitution des particules protéiques précipitées amorphes sèches, préparées selon l'une quelconque des revendications 1 à 7, étant inférieure à 1000 mOsmol/kg, inférieure à 800 mOsmol/kg ou inférieure à 600 mOsmol/kg.

9. Procédé selon l'une quelconque des revendications précédentes, la force de glissement mesurée pour ladite solution aqueuse comprenant > 140 mg/ml de protéine formée lors de la reconstitution des particules protéiques précipitées amorphes sèches, préparées selon l'une quelconque des revendications 1 à 8, étant < 15 N lorsque la solution passe à travers une aiguille de seringue 27 G d'1/2 pouce standard à 100 $\mu$l/s à une température de 20°C.

10. Procédé selon l'une quelconque des revendications précédentes, lesdites particules protéiques précipitées amorphes sèches étant reconstituées à une concentration de la protéine supérieure à 180 mg/ml, supérieure à 220 mg/ml, supérieure à 260 mg/ml ou supérieure à 300 mg/ml.

11. Procédé selon l'une quelconque des revendications précédentes, lesdites particules protéiques précipitées amorphes sèches étant reconstituées à une concentration comprise entre 120 mg/ml et 350 mg/ml, ou comprise entre 180 mg/ml et 300 mg/ml.

12. Procédé selon l'une quelconque des revendications précédentes, le diluant pour reconstitution des particules protéiques précipitées amorphes sèches étant : de l'eau pour injection (WFI), de l'eau distillée, de l'eau déminéralisée, de l'eau stérile pour injection (SWFI), de l'eau bactériostatique pour injection (BWFI) ; et le diluant pouvant comprendre un ou plusieurs tampons, tensioactifs, stabilisants de sels ou mélanges de ceux-ci.

13. Procédé selon l'une quelconque des revendications précédentes, lesdites particules protéiques précipitées amorphes sèches étant reconstituées pour former une solution aqueuse par reconstitution centrifuge.

14. Procédé selon la revendication 13 comprenant :

i) le transfert de particules protéiques précipitées amorphes sèches dans une récipient de reconstitution approprié, ou la préparation de particules protéiques précipitées amorphes sèches dans un récipient de reconstitution approprié ;
ii) l'ajout d'une quantité appropriée de diluant aqueux dans le récipient de reconstitution ;
iii) la centrifugation du récipient de reconstitution à une force centrifuge relative appropriée pendant une durée suffisante pour obtenir une reconstitution complète ou presque complète de la formulation de particules protéiques précipitées amorphes sèches dans le diluant aqueux et pour produire une solution protéique qui présente un moussage minimal ou inexistant.

15. Procédé selon la revendication 13, ledit diluant pour reconstitution étant une solution de la même protéine, ou un mélange de protéines tel qu'il est utilisé dans l'étape (i).

FIGURE 1

**EP 2 793 863 B1**

REFERENCES CITED IN THE DESCRIPTION

**Patent documents cited in the description**

- WO 2008132439 A2
- WO 2004062560 A2